# EUROPEAN PATENT APPLICATION

(11) **EP 4 151 252 A1**
(43) Date of publication of application: **22.03.2023**
(21) Application number: 21198028.9
(22) Date of filing: 21.09.2021
(51) Int. Cl.: A61M 5/158, A61B 17/34, A61M 25/01, A61M 25/06

(54) **NEEDLE SYSTEM HAVING CLOSED STATE AND OPEN STATE**

(71) Applicant: Xaga Surgical AB, 256 67 Helsingborg (SE)
(72) Inventor: FORSVALL, Andreas, 256 67 HELSINGBORG (SE); UVNÄS, Krister, 247 35 SÖDRA SANDBY (SE); EDBERG, Mats, 241 37 ESLÖV (SE); NORENSTAM, Rickard, 246 32 LÖDDEKÖPINGE (SE); CARMONIUS, Ola, 214 36 MALMÖ (SE)
(74) Representative: Brann AB

(57) **Abstract**

A needle system (10) for injection and aspiration of fluids through a subcutaneous venous port (1) comprises a main body (120) comprising a cavity (121), a conduit (101) with a proximal (211) and distal end (212), a needle section (102) with a proximal (221) and distal end (222), the distal end having an opening (203). The system comprises a rod (103) with a proximal (231) and distal end (232) and a length that is greater than the length of the needle section. The rod comprises an elongated rod shaft portion (106) configured to fit inside the needle section and a rod tip portion (104), at the distal end of the rod. The system comprises a rod control assembly (130). The distal end of the conduit is arranged in the main body and open to the cavity. The proximal end of the needle section is connected to the main body and open to the cavity. The shaft portion is inside the needle section and the proximal end of the rod is attached to the rod control assembly. The rod control assembly is configured to shift the rod in the proximal and distal direction to put the system into a closed state and into an open state. The rod tip portion blocks the distal end opening of the needle section in the closed state, and the rod tip portion is shifted distally relative to the needle section and unblocks the distal end opening of the needle section in the open state, thereby allowing fluid to be injected and/or aspirated through the conduit and the needle section in the open state.

## Description

### TECHNICAL FIELD

Embodiments herein relate to a needle system having an open state and a closed state for injection and aspiration through a subcutaneous vein port.

### BACKGROUND

Within the field of medicine, needle systems or needle arrangements for various purposes exist, for example needle arrangements for injection and aspiration. Injection and/or aspiration may take place directly into and out of a vein of a patient. Injection and/or aspiration may also take place via a subcutaneous venous port. In such cases a so-called Huber needle is often used. A Huber needle can penetrate the membrane of the subcutaneous venous port without cutting out parts of the membrane, which means that the size/mass of the membrane is retained, with the advantage that leakage can be avoided and thereby enabling a long lifetime of the subcutaneous port. Furthermore, avoidance of cutting off parts of the membrane means a reduced risk of unwanted particles entering the bloodstream of a patient.

Nevertheless, Huber needles are associated with drawbacks in that they may carry an undesired number of bacteria when penetrating the skin of the patient and thereby infecting the patient.

### SUMMARY

In view of the above, an object of the present disclosure is to overcome drawbacks related to prior art needle systems for injection and aspiration.

Such an object is achieved by a needle system for injection and aspiration through a subcutaneous vein port that comprises a main body comprising a cavity, a conduit having proximal end and a distal end, the conduit being arranged along a first longitudinal direction, a needle section having proximal end and a distal end, the distal end having a distal end opening, and the needle section being arranged along a second longitudinal direction, the second longitudinal direction being different than the first longitudinal direction. The needle system further comprises a rod having a proximal end and a distal end and a length that is greater than the length of the needle section. The rod comprises an elongated rod shaft portion configured to fit inside the needle section and a rod tip portion located at the distal end of the rod. The needle system further comprises a rod control assembly.

The distal end of the conduit is arranged in the main body and open to the cavity. The proximal end of the needle section is connected to the main body and open to the cavity. The rod is arranged such that the rod shaft portion is inside the needle section and the proximal end of the rod is attached to the rod control assembly. The rod control assembly is configured to shift the rod in the proximal and distal direction to put the needle system into a closed state and into an open state. The rod tip portion blocks the distal end opening of the needle section in the closed state and the rod tip portion is shifted distally relative to the needle section and unblocking the distal end opening of the needle section in the open state. Fluid is thereby allowed to be injected and/or aspirated through the conduit and the needle section in the open state.

In some embodiments, the rod control assembly may be configured to provide a proximally directed force on the rod biassing the needle system in the closed state.

Such a needle system retains the benefits of prior art, e.g. Huber type, needle systems while providing an advantage in terms of an even further reduced risk of bacteria accompanying the needle during insertion (typically as a consequence of poorly cleansed skin or the presence of crypts in the skin after previous punctures) through the skin of a person into a subcutaneous venous port. In fact, it has been found that some 5% of subcutaneous venous ports today become infected with mainly skin bacteria via skin punctures. Although some infections can be cured by use of antibiotics, in many cases the subcutaneous venous port needs to be urgently surgically removed, with the consequence that an ongoing treatment (often chemotherapy for cancer) must be discontinued. By noting that the rod tip portion blocks the distal end opening of the needle section in the closed state, the needle system of the present disclosure is a means of avoiding or at least minimizing the risk of infections and thereby minimizing the risk of being forced to discontinue treatment of a patient. This advantageous effect is further accentuated in embodiments where the rod control assembly is configured to provide a proximally directed force on the rod biassing the needle system in the closed state.

Moreover, since the needle system is configured with conduit and needle section along different directions, a needle system is thereby provided that can be arranged in a spatially advantageous manner in relation to a subcutaneous venous port. For example, an essentially perpendicular relationship between the conduit and the needle section enables a syringe or other injection/aspiration means connected to the conduit to be arranged along the body of a patient and thereby, e.g., minimizing the risk of injury due to disruption of the needle system when in use. The different longitudinal directions of the conduit and the needle section also enables a favourable arrangement of the rod control assembly. Since the needle system sometimes is attached to a person during several days, it is very practical that the longitudinal directions of the conduit and the needle section are such that a minimal protrusion from the body of the person can be obtained, thereby increasing the comfort, and minimizing the risk of dislocating the needle system from the person.

The rod control assembly may be arranged at least partly within the main body and/or the rod control assembly may be arranged at least partly outside the main body.

In some embodiments, the rod control assembly is configured to shift the rod in the proximal and distal direction by means of a mechanical system that is configured to convert a rotating motion of a first rod control assembly part around an axis along the second longitudinal direction into a shifting motion proximally and distally along the second longitudinal direction.

An advantageous effect of any such embodiment is that an operator of the needle system will find that it is practical and intuitive to use, it is compact and does not take up much space. It is also easy to configure the control assembly such that it is lockable in an open and in a closed position, and it is also easy to provide parts of the control assembly with visual markings that indicate that needle system is open or closed.

In some embodiments, having at least similar advantageous effects as for the embodiments summarized above, the rod control assembly is configured to shift the rod in the proximal and distal direction by means of a mechanical cam assembly that is configured to convert a rotating motion of a first cam assembly part into a shifting motion proximally and distally along the second longitudinal direction.

The needle section may in various embodiments comprise an outside surface coating for providing a desired friction between the needle section and skin and/or between the needle section and the subcutaneous vein port during insertion into the subcutaneous vein port.

For example, some of these embodiments provide an additional advantage in that a surface coating that provides a minimal friction also provides an advantageous effect in that it minimizes the adhesion of bacteria. Such an advantage is further appreciated in situations where the needle system is attached to the skin of a person during long periods of time, e.g. several days.

In some embodiments, the rod tip portion is configured with a plurality of facets that create cutting edges. Such embodiments have advantageous effects in that cutting edges that facilitate insertion into the subcutaneous vein port in may be combined with an appropriate surface friction between the needle section and the skin to minimize bacteria transport into the subcutaneous vein port.

These and further embodiments of a needle system, having the same and additional effects and advantages as those summarized above, will be described in the following detailed description.

### BRIEF DESCRIPTION OF THE DRAWINGS

Figure 1a schematically illustrates a needle system in a closed state,
figure 1b schematically illustrates the needle system of figure 1 a in an open state,
figure 1c schematically illustrates alternative configurations of a needle system,
figures 1d and 1e schematically illustrate a distal end of a needle and a rod of a needle system in a closed and an open state, respectively,
figure 1f schematically illustrates a rod tip portion,
figures 2a and 2b schematically illustrate a respective perspective view and a cross-sectional view of a needle system in a closed state,
figures 2c and 2d schematically illustrate a respective perspective view and a cross-sectional view of the needle system illustrated in figures 2a-b in an open state,
figures 3a-c schematically illustrate perspective views of a respective part of the needle system illustrated in figures 2a-d,
figures 3d-f schematically illustrate cross-sectional views of the respective parts of the needle system illustrated in figures 3a-c,
figures 4a and 4b schematically illustrate a respective perspective view and a cross-sectional view of a needle system in a closed state,
figures 4c and 4d schematically illustrate a respective perspective view and a cross-sectional view of the needle system illustrated in figures 2a-b in an open state, and
figures 5a and 5b schematically illustrate a needle system with a tip guard.

### DETAILED DESCRIPTION

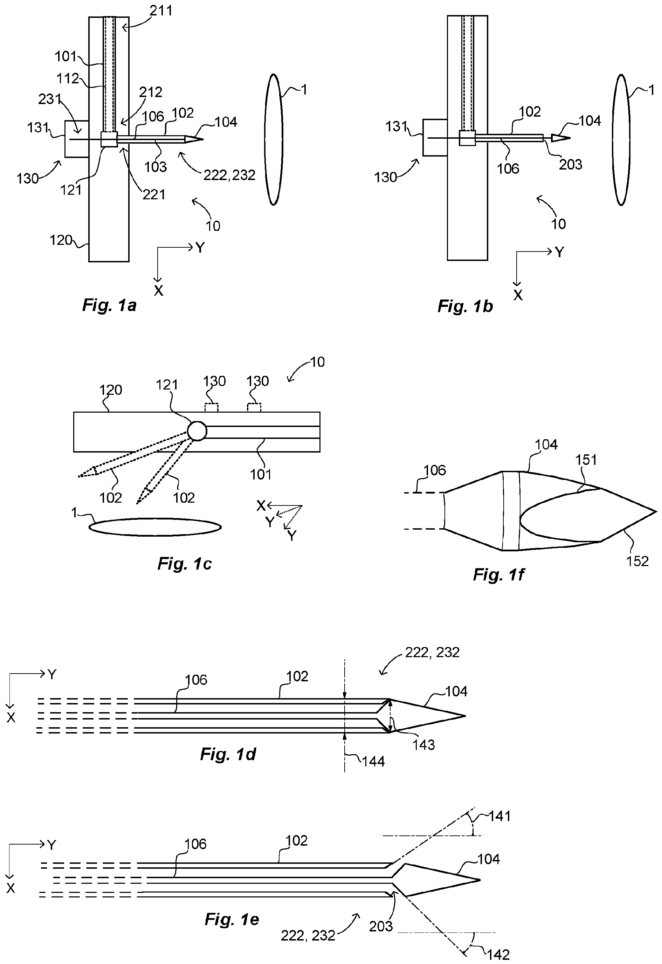

The needle system 10 schematically illustrated in figure 1a and figure 1b is configured for injection as well as for aspiration of fluids through a subcutaneous venous port 1 as discussed above. The needle system 10 comprises a main body 120 comprising a cavity 121 and a conduit 101 that has a proximal end 211 and a distal end 212, a needle section 102 that has a proximal end 221 and a distal end 222, the distal end 222 having a distal end opening 203. The needle system 10 further comprises a rod 103 that has a proximal end 231 and a distal end 232 and a length that is greater than the length of the needle section 102. Furthermore, the rod 103 comprises an elongated rod shaft portion 106 configured to fit inside the needle section 102 and a rod tip portion 104, located at the distal end 232 of the rod 103. The needle system 10 further comprises a rod control assembly 130.

The distal end 212 of the conduit 101 is arranged in the main body 120 and open to the cavity 121. The proximal end 221 of the needle section 102 is connected to the main body 120 and open to the cavity 121. The rod 103 is arranged such that the rod shaft portion 106 is inside the needle section 102 and the proximal end 231 of the rod 103 is attached to the rod control assembly 130.

The rod control assembly 130 is configured to shift the rod 103 in the proximal and distal direction to put the needle system 10 into a closed state as illustrated in figure 1a and into an open state as illustrated in figure 1b. The rod tip portion 104 blocks the distal end opening 203 of the needle section 102 in the closed state, and the rod tip portion 104 is shifted distally relative to the needle section 102 and unblocks the distal end opening 203 of the needle section 102 in the open state, thereby allowing fluid to be injected and/or aspirated through the conduit 101 and the needle section 102 in the open state.

The rod tip portion 104 may, as illustrated herein, have a general conical shape with a tip located distally, although other pointed shapes are possible as the skilled person will realize.

As illustrated in figures 1d and 1e, the rod tip portion 104 and the distal end 222 of the needle section 102 may in various embodiments be configured in a manner that has an effect of minimizing friction in relation to the subcutaneous venous port 1 or tissue through which the distal end 222 of the needle section 102 penetrates when in use, in particular during retraction out from the subcutaneous venous port 1. By minimizing friction during retraction, the risk of unintended opening of the needle section 102 is minimized. The rod tip portion 104 may in such embodiments be configured such that its diameter 143 at the distal end opening 203 of the needle section 102 is less than or equal to the outer diameter 144 of the needle section 102 at the distal end opening 203. Further minimizing of friction during retraction may be obtained by configuring the distal end opening 203 with an opening edge angle 141 that is equal or less than an angle 142 of the proximal end of the rod tip portion 104 that abuts the distal end opening 203 when the needle system 10 is in the closed state.

As illustrated in figure 1f, the rod tip portion 104 may in various embodiments be configured with a plurality of facets 151 that create cutting edges 152. Such configurations provide an advantageous effect in that less force is needed to insert the needle section 102 through tissue into the subcutaneous venous port 1 and thereby minimizing patient discomfort. Furthermore, such configurations minimize wear on the subcutaneous venous port 1 itself in that little or no material is scraped off the subcutaneous venous port 1 during insertion of the needle section 102. The number of facets 151 may be three, with a corresponding number of cutting edges 152, although the rod tip portion 104 may be configured with other numbers of facets 151. As exemplified in figure 1f, the rod tip portion 104 may be configured such that is a combination of a conical shaped tip portion with facets.

With regard to all embodiments herein, it is possible to provide a desired friction between the needle section 102 and skin and /or between the needle section 102 and the subcutaneous venous port 1 during insertion into the subcutaneous venous port 1. Low friction is desirable in order to minimize the force needed to insert the needle section 102, while high friction is desirable in order to minimize the amount of bacteria entering the subcutaneous venous port 1, i.e. bacteria is scraped off at skin level during insertion. The level of friction may be controlled by configuring at least the needle section 102 with a surface coating having appropriate friction characteristics. In any case, a smooth outer surface on the needle section 102 is advantageous in that it minimizes the risk of adhesion of bacteria during insertion of the needle section 102 into the subcutaneous venous port 1. Furthermore, by combining a low friction needle section 102 with a tip portion 104 that provides a relatively higher friction force in relation to tissue during insertion an advantageous effect is obtained in that a minimum amount of bacteria is carried into the subcutaneous venous port 1 during the insertion. For example, an uncoated tip portion 104, with or without facets, provides a relatively higher friction force in relation to tissue during insertion.

The conduit 101 is arranged along a first longitudinal direction X and the needle section 102 is arranged along a second longitudinal direction Y. As illustrated, the second longitudinal direction Y is different than the first longitudinal direction X and, for example, the first longitudinal direction X may be essentially perpendicular to the second longitudinal direction Y as illustrated in figures 1a an 1b. However, as illustrated in figure 1c, other angular relations between the first longitudinal direction X and the second longitudinal direction Y are also possible.

The conduit 101 may be a passage integrally formed in the main body 120. In other embodiments, as indicated in figures 1a and 1b by a dashed contour, the conduit 101 may comprise a second needle section 112. Although not illustrated, such a second needle section 112 may extend outside of the main body 120 and, in such embodiments, the conduit 101 may be considered as an interface between the cavity 121 and the second needle section 112.

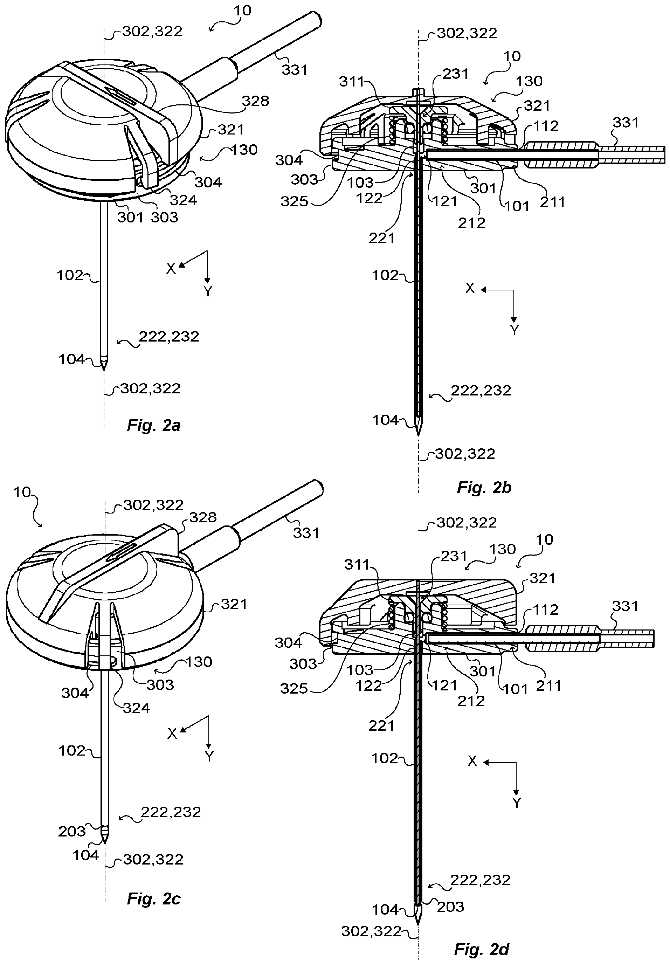

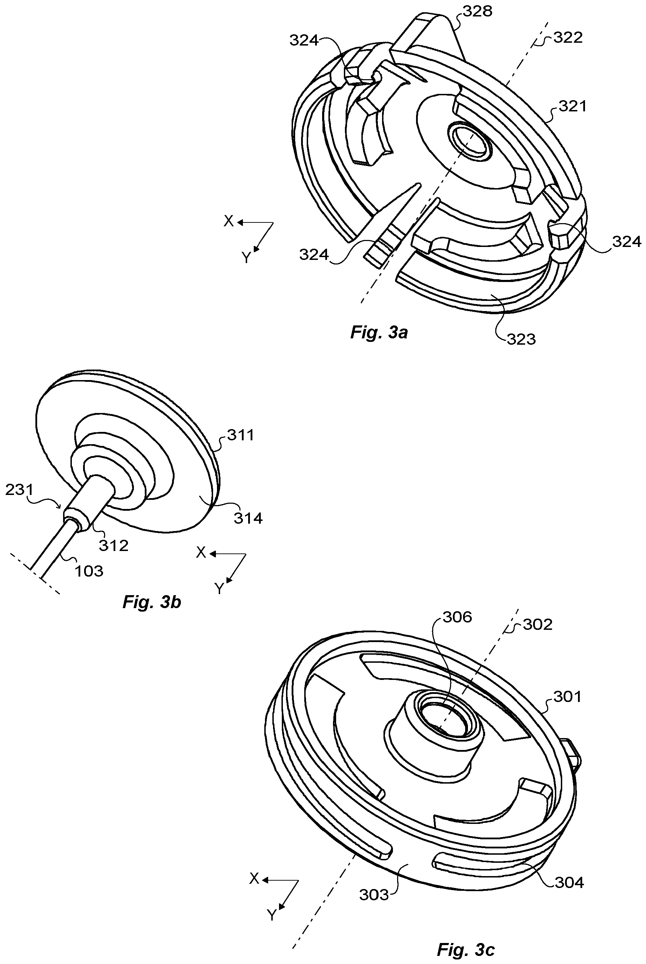

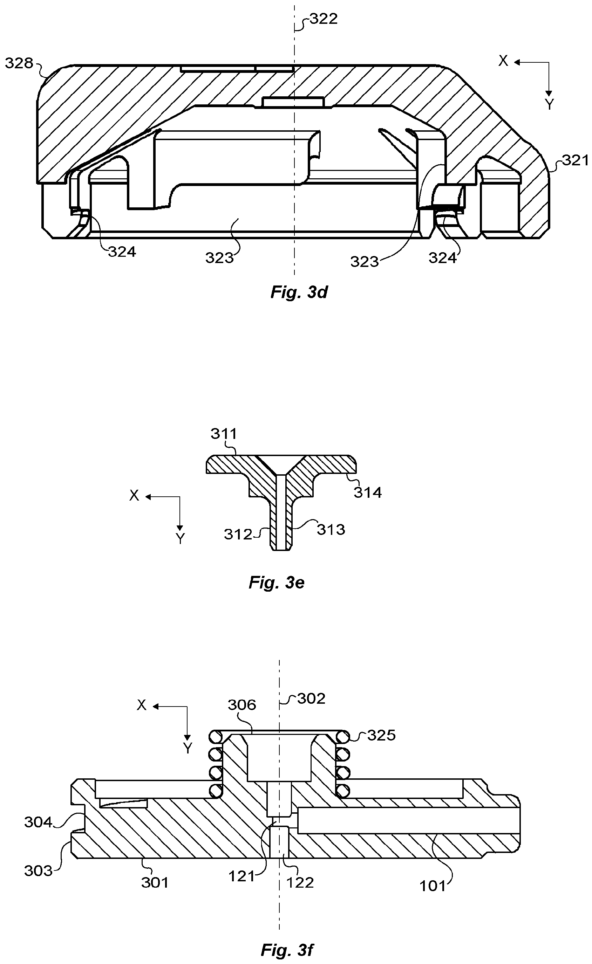

The rod control assembly 130 may be configured to shift the rod 103 in the proximal and distal direction by means of a mechanical system that is configured to convert a rotating motion of a first rod control assembly part 131 around an axis along the second longitudinal direction Y into a shifting motion proximally and distally along the second longitudinal direction Y. Such embodiments are illustrated very schematically in figure 1a-c. A more detailed embodiment of such a rotation/translation configuration will be described below in connection with figures 2a-d and 3a-f.

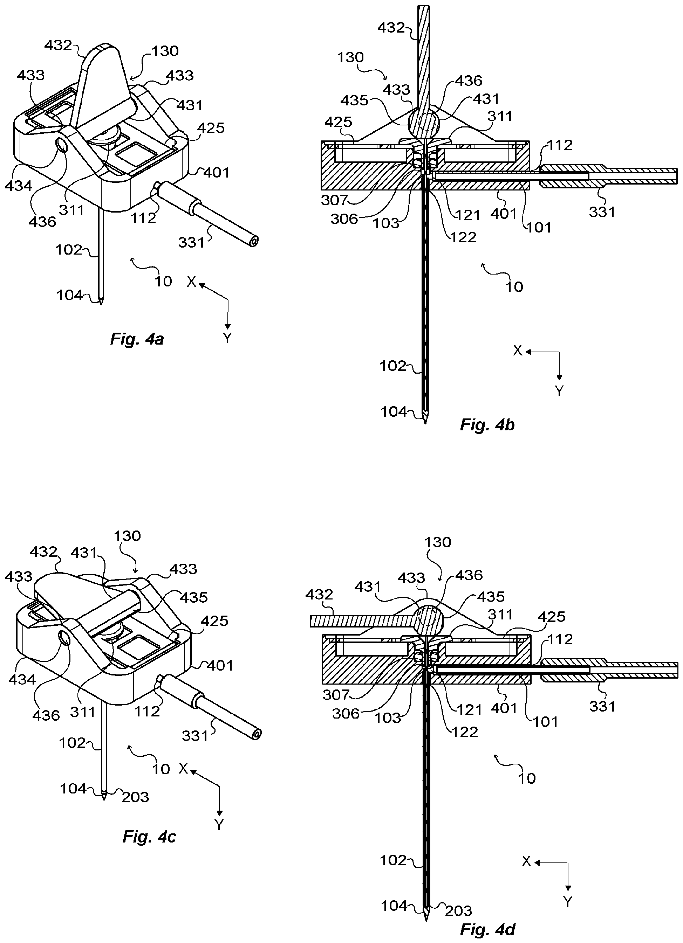

Other embodiments, also very schematically illustrated by figures 1a-c, as will be described in more detail below in connection with figures 4a-d. In such embodiments, the rod control assembly 130 may be configured to shift the rod 103 in the proximal and distal direction by means of a mechanical cam assembly that is configured to convert a rotating motion of a first cam assembly part into a shifting motion proximally and distally along the second longitudinal direction Y.

In any embodiment, the control assembly 130 may be configured to provide a proximally directed force on the rod 102 biassing the needle system 10 in the closed state.

Turning now to figures 2a-d and 3a-f, and with continued reference to figures 1a-c, an embodiment of the needle system 10 will be described in some more detail.

The needle system 10 illustrated in figures 2a-d and 3a-f comprises a main body 301 with a cavity 121, a conduit 101, a needle section 102, a rod 103 and a rod control assembly 130.

The main body 301 is circular cylindric with a longitudinal axis 302 and it is configured to be arranged with the longitudinal axis 302 along the second direction Y and it comprises a circumferential surface 303 that comprises a groove 304 having a gradient the direction of the longitudinal axis 302. As indicated in figure 3c, the groove 304 may encompass nearly the full circumference of the main body 301. However, the extension of the groove 304 along the circumferential surface 303 of the main body 301 may alternatively be shorter and/or divided into separate groove sections.

The conduit 101 along the first direction X opens up to the cavity 121 and a needle connecting recess 122 is configured such that the needle section 102 can be fitted and open up to the cavity 121. A rod holder recess 306 allows room for a rod holder 311 and also for sealing means (not illustrated) for sealing the cavity 121 from the rod holder recess 306. A section of a tube 331 is connected to a second needle section 112 that forms part of the conduit 101. Injection and/or aspiration takes place via the tube 331.

The rod control assembly 130 comprises a rod holder 311 and a cap 321. As such, the rod control assembly 130 is arranged at least partly within the main body 301 and at least partly outside the main body 301, and the cap 321 forms the first rod control assembly part 131 described above. The cap 321 comprises a circular cylindric inside surface 323 with a longitudinal axis 322 and the inside surface 323 of the cap 321 comprises at least one protrusion 324. The cap 321 is configured to be arranged with the longitudinal axis 322 along the second direction Y and configured to be arranged in relation to the main body 301 such that the inside surface 323 of the cap 321 encloses the circumferential surface 303 of the main body 301 and such that the at least one protrusion 324 fits within the groove 304.

Although the cap 321 in the embodiments illustrated in figures 2a-d and 3a-f comprises three protrusions 324, other numbers of protrusions are possible as long as a rotating motion of the cap 321 can ensure a proximal and distal motion of the cap 321 along the second direction Y when the protrusions 324 are forced by the gradient of the groove 304 in the main body 301 to move during rotation of the cap 321 with respect to the longitudinal axis 322. Although not illustrated in the drawings, the cap 321 and the main body 301 may be configured with a further small protrusion/recess combination that can interact and snap into each other at a certain angular relationship between the cap 321 and the main body 301. Such a protrusion/recess combination thereby provides a locking effect that prevents the cap 321 from inadvertent rotation.

In order to facilitate manual rotation of the cap 321, the cap 321 is provided with a grip 328 protruding from the outer surface of the cap 321. Needless to say, the grip 328 may have other configurations as long as the grip 328 enables a user to obtain a secure grip and thereby facilitates rotation of the cap 321.

The rod holder 311, comprising a holding part 312 and a flange part 314, is arranged between the main body 301 and the cap 321 and configured to hold the proximal end 231 of the rod 103 via the bore 313 in the holding part 312. For example, the proximal end 231 of the rod 103 may be fixed by means of glue, which is desirable in embodiments where the rod holder 311 is made of plastic and the rod 103 may be fixed by means of clamping in embodiments where the rod holder 311 is made of metal.

Although the rod holder 311 is exemplified as having a generally circular symmetry with respect to the longitudinal axis 302 of the main body 301 and the longitudinal axis 322 of the cap 321, other non-circular symmetrical shapes of the rod holder 311 are possible. Of course, any such other shape of the rod holder 311 would then correspond to a similarly shaped rod holder recess 306 in the main body 301.

As illustrated in figures 2a-d, the needle section 102 fits in the needle connecting recess 122 in the main body 301 and the rod 103 within the needle section 102 extends through the cavity 121. Furthermore, the rod 103 held in the bore 313 by the holding part 312 of the rod holder 311 that fits, and is movable along the second direction Y, in the rod holder recess 306.

As illustrated in figures 2b, 2d and 3f, in order to provide a proximally directed force on the rod 102 biassing the needle system 10 in the closed state, the control assembly 130 is configured such that a coil spring 325 is arranged in relation to the rod holder 311 and the main body 301, wherein the coil spring 325 is arranged between the rod holder 311 and the main body 301. The spring 325 abuts the rod holder 311 via the flange part 314. When the cap 321 is rotated in a first rotational direction, the protrusions 324 of the cap 321 follow the groove 304 and due to the gradient of the groove 304 the cap 321 is forced distally and the cap 321 thereby provides a force in the distal direction that overcomes the spring force of the spring 325 and, as a consequence, the rod 103 together with its the tip portion 104 move in the distal direction and thereby create the end opening 203 that defines the open state of the needle system 10. When the cap 321 is rotated in a second rotational direction, the protrusions 324 of the cap 321 follow the groove 304 and due to the gradient of the groove 304 and, assisted by the spring force provided by the spring 325, the cap 321 moves in the proximal direction. As a consequence, the rod 103 together with its the tip portion 104 move in the proximal direction and thereby closes the end opening 203 setting the needle system 10 into the closed state.

Turning now to figures 4a-d, and with continued reference to figures 1a-c, another embodiment of the needle system 10 will be described in some more detail.

The needle system 10 illustrated in figures 4a-d comprises a main body 401 with a cavity 121, a conduit 101, a needle section 102, a rod 103, a rod holder recess 306, a needle connecting recess 122 and a rod control assembly 130. The rod control assembly 130 is configured to shift the rod 103 in the proximal and distal direction by means of a mechanical cam assembly that is configured to convert a rotating motion of a first cam assembly part 431 into a shifting motion proximally and distally along the second longitudinal direction Y.

Similar to the embodiments described above, the conduit 101 along the first direction X in the main body 401 opens up to the cavity 121 and the needle connecting recess 122 is configured such that the needle section 102 can be fitted and open up to the cavity 121. The rod holder recess 306 allows room for the rod holder 311 and also for sealing means 307 for sealing the cavity 121 from the rod holder recess 306. A section of a tube 331 is connected to a second needle section 112 that forms part of the conduit 101. Injection and/or aspiration takes place via the tube 331.

The rod control assembly 130 illustrated in figures 4a-d comprises a rod holder 311, as described in connection with figures 3b and 3e, and a cam 431. The cam 431 is rotatably arranged in relation to the main body 401 and the rod holder 311 is arranged between the main body 401 and the cam 431 and configured to hold the proximal end 231 of the rod 103. As such, the rod control assembly 130 is arranged at least partly within the main body 401 and at least partly outside the main body 401, and the cam 431 forms the first rod control assembly part 131 discussed above.

Although the rod holder 311 is exemplified as having a generally circular symmetry, other non-circular symmetrical shapes of the rod holder 311 are possible. Of course, any such other shape of the rod holder 311 would then correspond to a similarly shaped rod holder recess 306 in the main body 401.

As for the embodiments described above, the control assembly 130 is configured to provide a proximally directed force on the rod 103 biassing the needle system 10 in the closed state. As illustrated in figures 4a-d, to achieve such a biassing force, a leaf spring 425 is arranged in relation to the rod holder 311 and the main body 401, wherein the leaf spring 425 is arranged between the rod holder 311 and the main body 401.

Such a configuration is obtained by means of the main body 401 having opposing protrusions 433 configured with opposing circular recesses 434 into which a cam axle 436 is arranged. The cam 431 is attached to the cam axle 436. Alternatively, the cam 431 may be integrally formed with the cam axle 436. Moreover, although the cam 431 exemplified herein is in the form of a cylindrical body that has an extension parallel with the cam axle 436 and having an extension that is more or less the same as the extension of the cam axle 436, in other embodiments it is foreseen that the cam may have a relatively smaller extension than the cam axle 436 in the direction that is parallel with the cam axle 436.

Similar to the embodiments described above, in order to provide a proximally directed force on the rod 102 biassing the needle system 10 in the closed state as illustrated in figures 4a-b, the leaf spring 425 abuts the rod holder 311 via the flange part 314.

When the cam axle 436 is rotated, the cam 431 rotates from a first rotational state as illustrated in figures 4a-b to a second rotational state as illustrated in figures 4c-d. In the transition to the second rotational state, the cam 432 increases a force in the distal direction upon the rod holder 311 that overcomes the spring force of the leaf spring 425 and, as a consequence, the rod 103 together with its the tip portion 104 move in the distal direction and thereby create the end opening 203 that defines the open state of the needle system 10.

A reverse rotation of the cam axle 436 and the cam 431 from the second rotational state to the first rotational state decreases the force in the distal direction upon the rod holder 311 and the spring force of the leaf spring 425 forces the rod 103 together with its the tip portion 104 to retract in the proximal direction and thereby closes the end opening 203 returning the needle system 10 to the closed state.

In order to facilitate manual rotation of the cam 431, the cam 431 is provided with a lever 432 protruding from the outer surface of the cam 431. Needless to say, the lever 432 may have other configurations as long as the lever 432 enables a user to obtain a secure grip and thereby facilitates rotation of the cam 431.

Moreover, as exemplified in figures 4a-d, the cam 431 may be configured with flattened peripheral surface areas 435 that provide an effect of locking the cam in the first and second rotational states in order to prevent that an inadvertent pull or push on the lever 432 results in an undesired rotation of the cam 431.

Although the needle system 10 has been illustrated by the embodiments of figures 2a-d and 3a-f where rotational movement of a control assembly is converted into a distal/proximal motion of a rod tip, and by the embodiments of figures 4a-d where a cam assembly provides distal/proximal motion of a rod tip, other embodiments may involve similar functionality. For example, distal/proximal motion of a rod tip may be provided by suitably configured control assembly parts that operate similar to a twist action ball point pen.

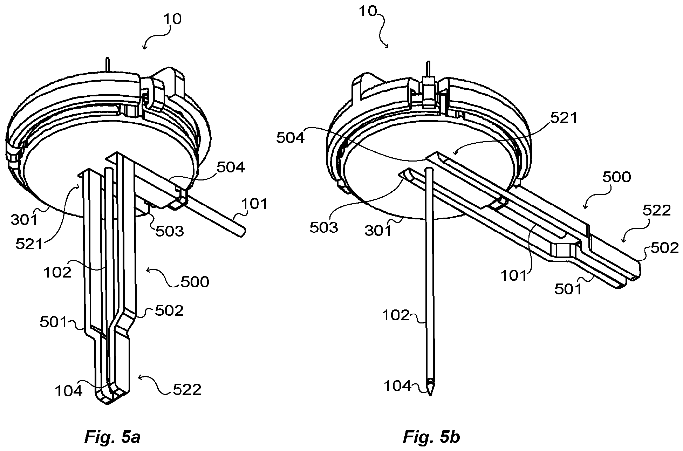

Now, with reference to all embodiments herein, as illustrated in figures 5a and 5b using the embodiment of the needle system 10 of figures 2a-d and 3a-f as an example, the needle system 10 may comprise an elongated tip guard 500 having a proximal end 521 and a distal end 522. The proximal end 521 is attached to the main body 301 and the tip guard 500 is configured to be set in a guarding state in which the distal end 522 of the tip guard 500 at least partly encloses the rod tip portion 104. An advantageous effect of such configuration is that it provides the needle system 10 with a safety function for avoiding injuries to a user of the needle system 10.

As illustrated and exemplified in figure 5a, the tip guard 500 may comprise a first elongated section 501 and a second elongated section 502. The elongated sections 501, 502 are in this example blade or leaf shaped and they have spatial extensions and are arranged in relation to each other in the guarding state such that the rod tip portion 104, and here also the needle section 102, is partly enclosed by the elongated sections 501, 502. The proximal end 521 of the tip guard 500, and thereby also the proximal ends of the elongated sections 501, 502, are attached to the main body 301 such that the elongated sections 501, 502 may pivot from the guarding state into a non-guarding state as illustrated in figure 5b. The elongated sections 501, 502 are attached to the main body 301 in a respective recess 503, 504 such that when pivoted into the non-guarding state, the elongated sections 501, 502 become flush with the surface of the main body 301. This is advantageous in that it provides a more or less flat surface that facilitates adhering the needle system 10 to the skin of a user/patient on which the needle system 10 is used.

Furthermore, with reference to all embodiments herein, an appropriate choice of material for the various parts of the needle system is a suitable plastic material, although various parts may in some embodiments be made of metal such as exemplified by the rod holder 311 described above.

## Claims

1. A needle system (10) for injection and aspiration through a subcutaneous vein port (1), comprising:
- a main body (120) comprising a cavity (121),
- a conduit (101) having proximal end (211) and a distal end (212), the conduit (101) being arranged along a first longitudinal direction (X),
- a needle section (102) having proximal end (221) and a distal end (222), the distal end (222) having a distal end opening (203), and the needle section (102) being arranged along a second longitudinal direction (Y), the second longitudinal direction (Y) being different than the first longitudinal direction (X),
- a rod (103) having a proximal end (231) and a distal end (232) and a length that is greater than the length of the needle section (102), wherein the rod (103) comprises an elongated rod shaft portion (106) configured to fit inside the needle section (102) and a rod tip portion (104), located at the distal end (232) of the rod (103),
- a rod control assembly (130),
wherein:
- the distal end (212) of the conduit (101) is arranged in the main body (120) and open to the cavity (121),
- the proximal end (221) of the needle section (102) is connected to the main body (120) and open to the cavity (121),
- the rod (103) is arranged such that the rod shaft portion (106) is inside the needle section (102) and the proximal end (231) of the rod (103) is attached to the rod control assembly (130),
- the rod control assembly (130) is configured to shift the rod (103) in the proximal and distal direction to put the needle system (10) into a closed state and into an open state, the rod tip portion (104) blocking the distal end opening (203) of the needle section (102) in the closed state, and the rod tip portion (104) is shifted distally relative to the needle section (102) and unblocking the distal end opening (203) of the needle section (102) in the open state, thereby allowing fluid to be injected and/or aspirated through the conduit (101) and the needle section (102) in the open state.

2. The needle system (10) of claim 1, wherein:
- the first longitudinal direction (X) is essentially perpendicular to the second longitudinal direction (Y).

3. The needle system (10) of any of claims 1 to 2, wherein the rod control assembly (130) is arranged at least partly within the main body (120).

4. The needle system (10) of any of claims 1 to 3, wherein the rod control assembly (130) is arranged at least partly outside the main body (120).

5. The needle system of any of claims 1 to 4, wherein the conduit (101) is a passage integrally formed in the main body (120).

6. The needle system of any of claims 1 to 5, wherein the conduit (101) comprises a second needle section (112).

7. The needle system (10) of any of claims 1 to 6, wherein:
- the rod control assembly (130) is configured to shift the rod (103) in the proximal and distal direction by means of a mechanical system that is configured to convert a rotating motion of a first rod control assembly part (131) around an axis along the second longitudinal direction (Y) into a shifting motion proximally and distally along the second longitudinal direction (Y).

8. The needle system (10) of claim 7, wherein the rod control assembly (130) comprises a rod holder (311) and a cap (321), said cap (321) forming the first rod control assembly part (131),
wherein:
- the main body (120, 301) is circular cylindric with a longitudinal axis (302) and configured to be arranged with the longitudinal axis (302) along the second direction (Y) and comprising a circumferential surface (303) that comprises a groove (304) having a gradient the direction of the longitudinal axis (302),
- the cap (321) comprises a circular cylindric inside surface (323) with a longitudinal axis (322), the inside surface (323) comprising at least one protrusion (324), the cap (321) being configured to be arranged with the longitudinal axis (322) along the second direction (Y) and configured to be arranged in relation to the main body (301) such that the inside surface (323) of the cap (321) encloses the circumferential surface (303) of the main body (301) and such that the at least one protrusion (324) fits within the groove (304), and
- the rod holder (311) is arranged between the main body (301) and the cap (321) and configured to hold the proximal end (231) of the rod (103).

9. The needle system (10) of any of claims 1 to 6, wherein:
- the rod control assembly (130) is configured to shift the rod (103) in the proximal and distal direction by means of a mechanical cam assembly that is configured to convert a rotating motion of a first cam assembly part (431) into a shifting motion proximally and distally along the second longitudinal direction (Y).

10. The needle system (10) of claim 9, wherein the rod control assembly (130) comprises a rod holder (311) and a cam (431), said cam (431) forming the first rod control assembly part,
wherein:
- the cam (431) is rotatably arranged in relation to the main body (301)
- the rod holder (311) is arranged between the main body (301) and the cam (431) and configured to hold the proximal end (231) of the rod (103).

11. The needle system (10) of any of claims 1 to 10, wherein:
- the control assembly (130) is configured to provide a proximally directed force on the rod (102) biassing the needle system (10) in the closed state.

12. The needle system (10) of claim 11, comprising:
- a spring (325, 425) arranged in relation to the rod holder (311) and the main body (301), wherein:
- the spring (325, 425) is a coil spring (325) or a leaf spring (425) and wherein the spring (325, 425) is arranged between the rod holder (311) and the main body (301).

13. The needle system (10) of any of claims 1 to 12, wherein the needle section (102) comprises an outside surface coating for providing a desired friction between the needle section (102) and skin and/or between the needle section (102) and the subcutaneous vein port (1) during insertion into the subcutaneous vein port (1).

14. The needle system (10) of any of claims 1 to 13, wherein the rod tip portion (104) is configured with a plurality of facets (151) that create cutting edges (152).

15. The needle system (10) of any of claims 1 to 14, further comprising an elongated tip guard (500) having a proximal end (521) and a distal end (522), said proximal end (521) being attached to the main body (301) and wherein the tip guard (500) is configured to be set in a guarding state in which the distal end (522) of the tip guard (500) at least partly encloses the rod tip portion (104).
